(19) Europäisches Patentamt
European Patent Office
Office européen des brevets

(11) EP 3 339 246 A1

(12) EUROPEAN PATENT APPLICATION

(43) Date of publication:
27.06.2018 Bulletin 2018/26

(51) Int Cl.:
*C01B 25/40* *(2006.01)* *C09K 21/00* *(2006.01)*

(21) Application number: 17210322.8

(22) Date of filing: 22.12.2017

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD TN

(30) Priority: 22.12.2016 CN 201611200685

(71) Applicant: Hangzhou JLS Flame Retardants Chemical Co., Ltd.
310011 Hangzhou (CN)

(72) Inventor: The designation of the inventor has not yet been filed

(74) Representative: Uexküll & Stolberg
Partnerschaft von
Patent- und Rechtsanwälten mbB
Beselerstraße 4
22607 Hamburg (DE)

# (54) METAL ION MODIFIED MELAMINE POLYPHOSPHATE COMPOUND, PROCESS FOR ITS PREPARATION AND USE OF IT

(57) This invention concerns a melamine polyphosphate salt compound modified by a metal ion of the following formula. The formula is as shown in formula (I):

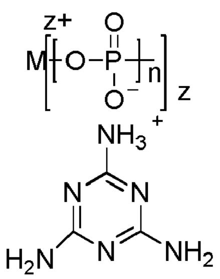

(I)

with M: metal ion, $Z^+$ is the oxidation state of M, n is average degree of polymerization of polyphosphoricacid , n≥2. This invention of a metal ion modified melamine polyphosphate has stable heat resistance, excellent flame retardancy and can be easily incorporated into various plastics. This invention application also reveals the process method which includes following steps: (1) add polyphosphoric acid slowly into melamine while stirring and then increase the temperature for reaction and made the compound of melamine polyphosphate. (2) then add metal ion into the melamine polyphosphate compound. Stir and then increase temperature for reaction and made the final product. It's a facile process, stable and controllable, with a mild reaction temperature. Other advantages are low energy consumption, less environmental pollution and no corrosion of the reactor tubes.

Figure 1

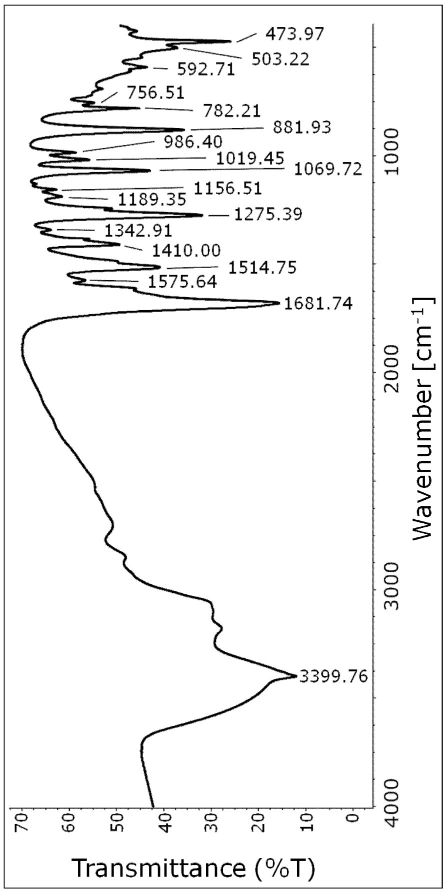


EP 3 339 246 A1

## Description

### Industry Area for the Technology

The present invention is directed to a metal ion modified melamine polyphosphate compound with increased high heat stability, a process for its preparation as well as its use.

### Background of the Technology

Compared with previously known halogenated flame retardant compounds the flame retardant compound of the present invention is non halogenated, non toxic and environmentally friendly.

Gordon L. Nelson in the book, Fire and Polymers, 5/9/1990, pp. 228-231, explains in detail how melamine polyphosphate is synthesized by reacting melamine and phosphoric acid:

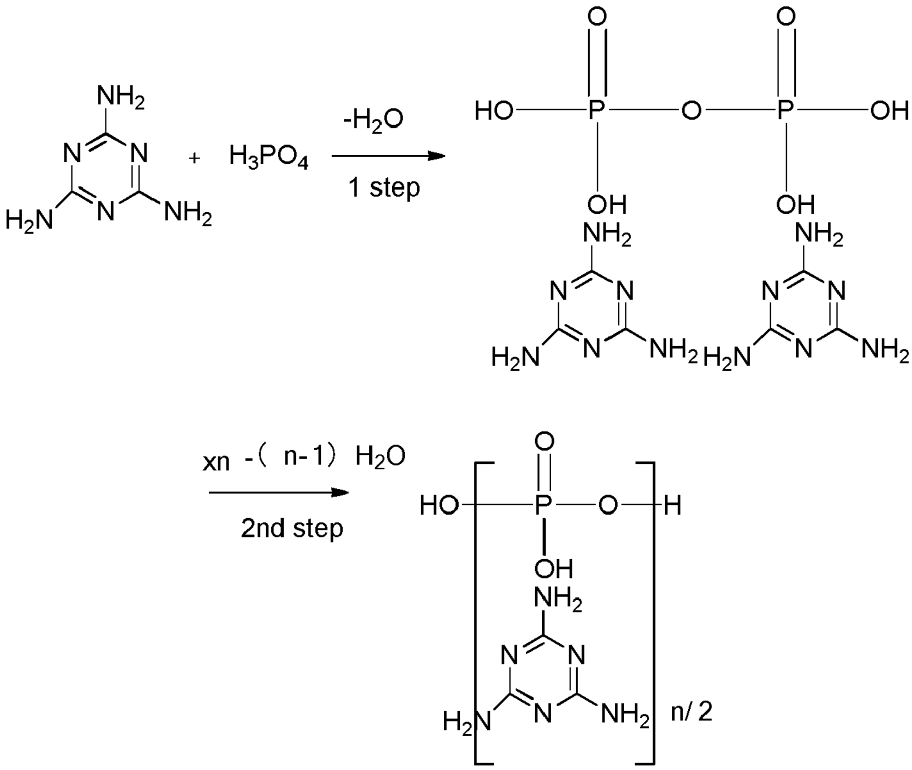


An article published on May 31st 2003 in Ind. Eng. Chem. Res. entitled "Polyphosphate Flame Retardants with Increased Heat Resistance" is a review of methods for using orthophosphoric acidic polycondensation reactions at high temperatures in order to obtain melamine polyphosphate. The PH value, water solubility, TGA decomposition temperature, and the ratio of melamine to phosphorus are reported.

The Chinese patents CN 101014645A and CN 1314898A describe the use of melamine and orthophosphoric acid to synthesize melamine orthophosphates. A polycondensation reaction is conducted in an ammonia atmosphere at high temperature to obtain melamine polyphosphate. This method requires high energy consumption, long reaction times, and suffers from equipment corrosion issues.

WO 9744377 and CN 13624138 describe the use of melamine, orthophosphoric acid and urea to synthesize a complex salt at low temperature, which is then condensed to melamine polyphosphate at high temperature.

EP 1789475B1 introduces another method by synthesizing a metal polyphosphate salt first which is then reacted with the melamine. This method gives a compound with high metal content and a low phosphorus content and, therefore, with a lower degree of fire retardancy.

CN 1733778A also uses a metal ion to modify melamine polyphosphate. However, the reaction takes place in the presence of a solvent. This requires a more complicated three step process that gives a less pure compound.

The disadvantages of the above process methods can be summarized as follows:

1. The pH of the final product is relatively low and the final product includes the contaminant residue P-OH. Therefore,

when used in polyamide and polyester the heat stability will be too low.
Furthermore, the P-OH residue will affect the mechanical properties negatively.

2. The production equipment must be very advanced and complicated to meet the product specifications. Furthermore, the high production temperature will produce an orthophosphoric acid vapor and the urea decomposition will produce ammonia gas.

3. The heat stability will be too low with a starting decomposition temperature of 310 °C and after compound decomposition the acidity will be high. The acidity will decompose the nylon resulting in reduced mechanical properties.

Melamine polyphosphate salts have been widely used as flame retardant additives. Gordon L. Nelson in the book Fire and Polymers (May 9th, 1990) has described the synthesis method and applications of melamine and its salts. On page 211 it is described that "Melamine and its salts are widely used in formulations of fire retardant additives, particularly of the intumescent type".
In 2003, Ou Yu Xiang published an article in China Plastics entitled "Combustion Behavior and Thermal Pyrolysis of PA6 Flame Retarded with Melamine Polyphosphate Based Intumescent Flame Retardants". In this article he described the melamine polyphosphate salt used in the PA6 as well as detailed specifications.
In 1999, EP 1040754A2, as well as its corresponding US patent application 20070299171, discloses that melamine polyphosphate salts are synergistic with other phosphinates giving very good flame retardant efficiency. This combination has been widely used in the engineering thermoplastics, both polyester and polyamide, for electrical parts.

**The present invention**

The purpose of this invention is to solve the existing technical issues listed above. A new product with high heat stability and excellent flame retardant efficiency is provided that can be widely used in many applications. The present invention is directed to a metal ion modified melamine polyphosphate salt.
It has advantageously been found that the present invention provides a simple manufacturing process with mild reaction conditions that can be readily controlled, consumes less energy, gives less environmental pollution, and virtually no equipment corrosion.
In order to realize the above result this invention will adopt the following technical solutions.
This invention concerns a melamine polyphosphate salt compound modified by a metal ion of the following formula:

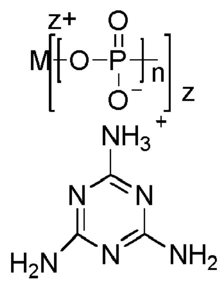

(I)

wherein M is a metal ion, $Z^+$ is the oxidation state of M, n is the average degree of polymerization of polyphosphoric acid with n≥2. The metal ion modified melamine polyphosphate according to the invention has stable heat resistance, excellent flame retardancy and can be easily incorporated into various plastics. Z is the oxidation state of M, the metal ion. Obviously, the value of Z will vary depending on the metal ion used. Preferably, M is selected from the group consisting of Al, Mg, Zn and mixtures thereof.
The present invention is also directed to a process for preparing the metal ion modified melamine polyphosphate salt, which process includes following steps:

(1) While stirring, polyphosphoric acid is added to melamine. Then the temperature is increased to 80° C to 300° C, and the resulting mixture is reacted for 1-10 hours. A white powder of the melamine polyphosphate salt precursor is obtained. This invention is a direct method of reacting polyphosphoric acid with melamine thereby eliminating the synthesis of a melamine phosphate precursor in order to obtain melamine polyphosphate. Furthermore, the product's degree of polymerization is higher when polyphosphoric acid is used as compared to the melamine phosphate route.

(2) A metal ion is added to the melamine polyphosphate precursor while mixing the reaction mixture is continued. After adding the metal ion the temperature is increased to 270 to 350° C. Then, the mixture is reacted for 0.5 to 5 hours. After that, the final product is obtained.

In step 1 the mol ratio between melamine and phosphorus is preferably (0.95-1.15) : 1.0.

Preferably, the typical degree of polymerization of polyphosphoric acid is 1.2 or higher ($n \geq 1.2$).

Preferably, the degree of polymerization of polyphosphoric acid is between 1.2 and 10.0.

According to step 2, the metal ion can preferably be added in the form of aluminum hydroxide, magnesium hydroxide, or zinc hydroxide or in the form of their oxides, such as one or more of alumina, magnesia and/or zinc oxide.

According to step 2, the mol ratio between the metal compound and melamine is preferably 1 to 10-20.

Furthermore, it is preferred that the final product of the process according to the invention, i.e. the metal ion modified polyphosphate salt compound according to the invention, has an average degree of polymerization of $n \geq 2$. Furthermore, the mol ratio between melamine and phosphorus preferably is preferably <1.09. Moreover, the pH of a test sample of the metal ion modified polyphosphate salt compound according to the invention is preferably between 4.5 to 5.0 at 25° C in a 10% suspension. Furthermore, the water solubility of the compound according to the invention is preferably <0.2 g/100g water. It is also preferred that its degradation temperature, at which the loss of weight amounts to 2 %, is >350° C. Furthermore, it is preferred that the content of metal ion amounts to 0.1 to 10% by weight %, based on the total weight of the compound. In addition, the phosphorus content is preferably 13.5 to 14.5 weight %, based on the total weight of the compound.

Accordingly, the metal ion modified melamine polyphosphate of the present invention is preferably characterized by one or more of the following:

average degree of polymerization of $n \geq 2$;

mol ratio between melamine and phosphorus of < 1.09;

pH of 4.5 to 5.0, preferably 4.5 to 4.9, more preferably 4.5 to 4.8 or 4.5 to 4.7, at 25 °C in a 10% suspension;

water solubility of <0.2 g/100 g water, preferably <0.15 g/100 g water, more preferably <0.10 g/100 g water at 25 °C;

degradation temperature at 2% weight loss of ≥350° C, preferably ≥355°C, more preferably ≥360°C;

content of metal ion of 0.1 to 10% by weight %, preferably 2 to 8 % by weight, more preferably 3 to 7 % by weight; and/or

phosphorus content of 13.5 to 14.5 weight %, preferably 13.8 to 14.3 weight %.

In particular, the metal ion modified melamine polyphosphate of the present invention preferably has a pH of 4.5 to 5.0, preferably 4.5 to 4.9, more preferably 4.5 to 4.8 or 4.5 to 4.7, as measured at 25 °C in a 10 wt.-% aqueous suspension of the compound of the invention.

This metal ion modified melamine polyphosphate compound can be used in both thermoplastic and thermoset resins as a flame retardant. Especially it can be used in the glass fiber reinforced PA6, PA66, PET, PBT, PETG and polyolefin.

Therefore, this invention has the following advantages:

(1) It provides a metal ion modified polyphosphate salt with high heat stability, excellent flame retardancy and application versatility.

(2) It provides a facile process method that is stable and controllable, with a mild reaction temperature. Other advantages are low energy consumption, less environmental pollution and virtually no corrosion of the reactor tubes.

**Figures**

Figure 1 shows an FTIR spectrum of the compound of Example 1, a metal ion modified melamine polyphosphate according to the invention.

Figure 2 shows an FTIR spectrum of commercially available melamine polyphosphate.

## Examples

The present invention is further described below by way of examples and with reference to the accompanying drawings.

### Example 1

(1) In step 1 a mol ratio between melamine and phosphorus of 0.95 to 1.0 was used. The degree of polymerization of polyphosphoric acid was n=1.2. While stirring, polyphosphoric acid was slowly added into the melamine. Then the temperature was increased to 80°C and the mixture was allowed to react for 10 hours. A white powder of melamine polyphosphate precursor was obtained.

(2) The mol ratio of the metal ion compound and melamine was 1 to 10. The metal ion compound was added to the melamine polyphosphate precursor. The metal ion was aluminum hydroxide. After mixing the temperature was increased to 270°C and the mixture was reacted for 5 hours to obtain the final product.

### Example 2

(1) In step 1 a mol ratio between melamine and phosphorus of 1.15 to 1.0 was used. The degree of polymerization of polyphosphoric acid was n=10. While stirring, polyphosphoric acid was slowly added into the melamine. Then the temperature was increased to 300° C and the mixture was allowed to react for 1 hour. A white powder of melamine polyphosphate precursor was obtained.

(2) The mol ratio of the metal ion compound and melamine was 1 to 20. The metal ion compound was added to the melamine polyphosphate precursor. The metal ion wass magnesium hydroxide. After mixing the temperature was increased to 350° C and the mixture was reacted for 0.5 hours to obtain the final product.

### Example 3

(1) In step 1 a mol ratio between melamine and phosphorus of 1.12 to 1.0 was used. The degree of polymerization of polyphosphoric acid was n=8. While stirring, polyphosphoric acid was slowly added into the melamine. Then the temperature was increased to 150° C and the mixture was allowed to react for 6 hours. A white powder of melamine polyphosphate precursor was obtained.

(2) The mol ratio of the metal ion compound and melamine wass 1 to 12. The metal ion compound was added to the melamine polyphosphate precursor. The metal ion was zinc hydroxide. After mixing the temperature was increased to 300° C and the mixture was reacted for 3.0 hours to obtain the final product.

### Example 4

(1) In step 1 a mol ratio between melamine and phosphorus of 1.1 to 1.0 was used. The degree of polymerization of polyphosphoric acid was n=5. While stirring, polyphosphoric acid was slowly added into the melamine. Then the temperature was increased to 280° C and the mixture was allowed to react for 3 hours. A white powder of melamine polyphosphate precursor was obtained.
(2) The mol ratio of the metal ion compound and melamine was 1 to 15. The metal ion compound was added to the melamine polyphosphate precursor. The metal ion was aluminum oxide. After mixing the temperature was increased to 320° C and the mixture was reacted for 3.0 hours to obtain the final product.

**Example 5**

(1) In step 1 a mol ratio between melamine and phosphorus of 1.15 to 1.0 was used. The degree of polymerization of polyphosphoric acid was n=7. While stirring,polyphosphoric acid was slowly added into the melamine. Then the temperature was increased to 250° C and the mixture was allowed to react for 3 hours. A white powder of melamine polyphosphate precursor was obtained.

(2) The mol ratio of the metal ion compound and melamine was 1 to 15. The metal ion compound was added to the melamine polyphosphate precursor. The metal ion was magnesium oxide. After mixing the temperature was increased to 290° C and the mixture was reacted for 4.0 hours to obtain the final product.

**Example 6**

(1) In step 1 a mol ratio between melamine and phosphorus of 0.95 to 1.0 was used. The degree of polymerization of polyphosphoric acid was n=2. While stirring, polyphosphoric acid was slowly added into the melamine. Then the temperature was increased to 220° C and the mixture was allowed to react for 6 hours. A white powder of melamine polyphosphate precursor was obtained.

(2) The mol ratio of the metal ion compound and melamine was 1 to 18. The metal ion compound was added to the melamine polyphosphate precursor. The metal ion was zinc oxide. After mixing the temperature was increased to 290° C and the mixture was reacted for 3.5 hours to obtain the final product.

**Example 7**

(1) In step 1 a mol ratio between melamine and phosphorus of 1.1 to 1.0 was used. The degree of polymerization of polyphosphoric acid was n=2. While stirring, polyphosphoric acid was slowly added into the melamine. Then the temperature was increased to 300° C and the mixture was allowed to react for 1 hour. A white powder of melamine polyphosphate precursor was obtained.

(2) The mol ratio of the metal ion compound and melamine was 1 to 20. The metal ion compound was added to the melamine polyphosphate precursor. The metal ion compound was a mixture of aluminum hydroxide and zinc oxide in a mol ratio of 1 to 1. After mixing the temperature was increased to 320° C and the mixture was reacted for 3.0 hours to obtain the final product.

**Example 8**

(1) In step 1 a mol ratio between melamine and phosphorus of 1.12 to 1.0 was used. The degree of polymerization of polyphosphoric acid was n=5. While stirring, polyphosphoric acid was slowly added into the melamine. Then the temperature was increased to 150° C and the mixture was allowed to react for 6 hours. A white powder of melamine polyphosphate precursor was obtained.

(2) The mol ratio of the metal ion compound and melamine was 1 to 15. The metal ion compound was added to the melamine polyphosphate precursor. The metal ion compound was a mixture of magnesium hydroxide and magnesium oxide in a mol ratio of 2 to 3. After mixing the temperature was increased to 300° C and the mixture was reacted for 3.5 hours to obtain the final product.

**Example 9**

(1) In step 1 a mol ratio between melamine and phosphorus of 1.0 to 1.0 was used. The degree of polymerization of polyphosphoric acid was n=3. While stirring, polyphosphoric acid was slowly added into the melamine. Then the temperature was increased to 280° C and the mixture was allowed to react for 3 hours. A white powder of melamine polyphosphate precursor was obtained.

(2) The mol ratio of the metal ion compound and melamine was 1 to 12. The metal ion compound was added to the melamine polyphosphate precursor. The metal ion compound was a mixture of zinc hydroxide, aluminum oxide and magnesium oxide in a mol ratio of 1 to 1 to 1. After mixing the temperature was increased to 280° C and the mixture was reacted for 3.2 hours to obtain the final product.

Properties of the metal ion modified polyphosphate salts obtained in the above examples are listed in Table 1 below:

**Table 1**

| Item | Phosphorus content % | 2%wt. loss temperature | pH | Water solubility, g/100g water |
|---|---|---|---|---|
| Example1 | 14.35 | 354 | 4.53 | 0.16 |
| Example2 | 13.96 | 357 | 4.81 | 0.12 |
| Example3 | 14.10 | 363 | 4.67 | 0.11 |
| Example4 | 14.21 | 362 | 4.51 | 0.07 |
| Example5 | 14.01 | 356 | 4.95 | 0.17 |
| Example6 | 14.39 | 371 | 4.53 | 0.09 |
| Example7 | 14.00 | 374 | 4.83 | 0.04 |
| Example8 | 13.90 | 368 | 4.87 | 0.05 |
| Example9 | 14.22 | 372 | 4.69 | 0.05 |

The phosphorous content was determined by applying the principle of polyphosphate salt decomposition into orthophosphate ions and subsequent reaction with Quimociac reagent in acidic medium to generate yellow phosphorous molybdate quinoline precipitation which is then used for phosphorus determination. In the analysis, unless specified otherwise, analytical reagents, destilled water or water of equal purity were used.

The Quimociac reagent was obtained via Solutions a to d.

Solution a: In a 400 ml beaker, dissolve 70 g of sodium molybdate in 150 ml of water.

Solution b: In a 1000 ml beaker, dissolve 60 g of citric acid in 85 ml of nitric acid solution and 150 ml of water.

Solution c: Under stirring, add Solution A into Solution B and mix.

Solution d: Add 35 ml of nitric acid solution and 5 ml of quinoline in 100 ml.

The Solution d was added to the Solution c, the resulting mixture was stirred, stood overnight and filtered (paper or cotton). The filtrate was added to 280 ml of acetone and diluted with water to 1000 ml. The mixture was stored in polyethylene bottles avoiding light and heat.

Sample decomposition and preparation:

Weigh the sample (about 0.2 to 0.3g; weighed to 0.0002g) in a 250 ml triangular beaker, add a small amount of water wet, add 20 ml of 20 g/l sodium hydroxide solution, add 100 ml of water, heat in a hot plate to micro-boiling and keep the micro-boiling for 40min. Add the obtained mixture into a certain amount of water, cool, add 30 ml of nitric acid (1+1), heat to micro-boil in a hot plate to maintain micro-boiling for 30 min, add some water, cool, transfer to 250 ml volumetric flask, dilute to the scale and mix well.

Determination:

Pipette a sample solution of 25.00 ml in a 250ml triangular beaker, add 10ml of 1 + 1 nitric acid solution, add 150 ml of water, placed in a hot plate to micro-boiling, add 35 ml of Quimociac reagent while it is hot, mix, cool, and shake the sample at least 3 times during in the process of cooling. Filter the mixture with a crucible filter (No. 4, 30 ml) previously dried at 180 ± 5 °C until constant weight. The supernatant was first filtered off and then washed by pouring for 1-2 times (about 15 ml of water each time). The precipitate was placed in a drying oven at 180 ± 5 ° C with the filter, after the temperature reached 180 °C. The precipitate was dried for 45 min, transferred to a desiccator, cooled and

weighed.

Calculation Results:

The sample in mass percentage of phosphorus content X was calculated according to equation (1):

$$X = [(m_1-m_0) \times 1400] / [m \times 25/250] = [(m_1-m_0) \times 14] / m \qquad (1),$$

wherein $m_0$ is the crucible filter quality (in g), $m_1$ is the mass of the crucible filter and the quality of phosphomolybdate quinoline precipitation (in g), and m is the quality of the sample in the testing (in g).

Allowable difference:

The absolute difference between parallel determinations should not exceed 0.5%. Take the arithmetic mean of parallel determination results as the measurement result.

The 2 wt.-% loss temperature is the temperature at which the polyphosphate salt shows a loss of weight of 2%. It was determined using a Mettler Toledo TGA/DSC1 with $N_2$ and a heating rate of 20°C/min.

The pH of a 10 wt.-% suspension of the metal ion modified polyphosphate salt compound in water was determined at 25°C in accordance with ISO6353-1-1982.

The water solubility was determined at 25°C by the following test procedure. In 90 g of water 10 g of the sample to be tested was added. The mixture was stirred for 30 min at the control solution temperature (25 ± 1.0°C) and was then transferred to a centrifuge tube. After separation by centrifugation (2000 r/min; rotation for 20 min), the upper clear liquid was removed and placed in a dry weighing bottle which was then dired in an electric drying oven at 160 ± 5 °C to constant weight. The solubility $X_2$ was calculated as follows:

$$X_2 = (m_2-m_0)/(m_1-m_0) \times 100,$$

wherein $m_0$ is the mass of the weighing bottle (in g), $m_1$ is the mass of the weighing bottle with upper clear liquid (in g) and $m_2$ is the mass of the weighing bottle of constant weight (in g).

From Table 1 it can be seen that the metal ion modified melamine polyphosphates show excellent heat stability and low water solubility.

The above listed metal ion modified melamine polyphosphates were processed on a twin screw extruder in accordance with the following formulations: metal ion modified melamine polyphosphate salt at 6 wt.% , DEPAL at 7 wt.%, anhydrous zinc borate at 2 wt.%, PA66 (BASF A3K) at 59.3 wt.%, glass fiber at 25 wt.%, antioxidant at 0.2 wt.%, other additives at 0.5 wt. %. The flame retardant properties of the obtained plastics are shown in the following Table 2.

**Table 2**

| Item | Flame Retardancy | |
|---|---|---|
| | Rating | LOI |
| 1 | 1.6mmV1 | 28 |
| 2 | 1.6mmV1 | 28 |
| 3 | 1.6mmV1 | 30 |
| 4 | 1.6mmV0 | 30 |
| 5 | 1.6mmV0 | 30 |
| 6 | 1.6mmV0 | 28 |
| 7 | 1.6mmV0 | 30 |
| 8 | 1.6mmV0 | 28 |
| 9 | 1.6mmV0 | 30 |

The flame retardancy rating was determined in accordance with the UL94 standard. The limiting oxygen index

(LOI) was determined in accordance with ISO 4589-2:1996.

It can be seen from Table 2 that the metal ion modified melamine polyphosphates according to the present invention have excellent flame retardancy properties.

Figure 1 shows an FTIR spectrum of the metal ion modified melamine polyphosphate obtained in Example 1.

Figure 2 shows an FTIR spectrum of commercially available melamine polyphosphate.

It can be seen from a comparison of these two spectra that the spectrum of Example 1 does not completely match the spectrum of the commercial melamine polyphosphate. The spectrum of Example 1 shows no strong absorption band at 3173 cm-1 of a N-H group. This demonstrates that the metal compound reacted with the active phosphorus hydroxyl groups to reduce theamino activity in the ortho-position resulting in a weakening or even disappearing of the corresponding N-H peak. This proves that the metal ion reacted with the melamine polyphosphate compound. The structure of Example 1 has been changed from that of Example 2, the commercial melamine polyphosphate. Therefore, the present invention results in a change in the chemical structure and the obtained product is a new compound. The above example is only one preferred embodiment of the invention. However, the invention is not limited to Example 1 or any other example. The above examples illustrate preferred embodiments of the present invention, but are not intended to limit the present invention in any form.

## Claims

1. Metal ion modified polyphosphate salt compound having a formulation as follows:

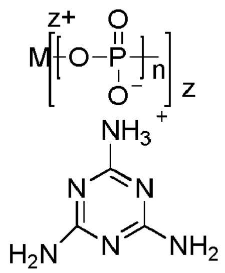

wherein M is a metal ion, $Z^+$ is the oxidation state of M, n is average degree of polymerization of polyphosphoric acid with $n \geq 2$.

2. Process for preparing the metal ion modified polyphosphate salt of claim 1 comprising:

(1) adding polyphosphoric acid slowly into melamine while stirring, then increasing the temperature to 80° C to 300° C, then reacting the mixture between 1-10 hours to obtain a white powder of melamine polyphosphate salt precursor;
(2) adding metal ion compound to the melamine polyphosphate precursor, then increasing the temperature to 270 to 350 °C and reacting the mixture for 0.5 to 5 hours to obtain the final product.

3. Process of claim 2, wherein the mol ratio between melamine and phosphorus is (0.95 to 1.15):1.0.

4. Process of claim 2 or 3, wherein the degree of polymerization of polyphosphoric acid is $n \geq 1.2$.

5. Process according to claim 4, wherein the degree of polymerization of polyphosphoric acid is between 1.2 to 10.0.

6. Process according to any one of claims 2 to 5, wherein the metal ion compound is a hydroxide and/or an oxide, with the hydroxide including one or more of aluminum hydroxide, magnesium hydroxide, and zinc hydroxide, and with the oxide including one or more of alumina, magnesium oxide and zinc oxide.

7. Process according to claim 2, wherein in step (2) the mol ratio between the metal compound and melamine is 1 : (10 to 20).

8. Process according to claim 2, wherein the metal ion modified melamine polyphosphate is **characterized by** one or more of the following:

average degree of polymerization of n≥2;
mol ratio between melamine and phosphorus of < 1.09;
pH of 4.5 to 5.0, preferably 4.5 to 4.9, more preferably 4.5 to 4.8 or 4.5 to 4.7, at 25° C in 10% suspension;
water solubility of <0.2 g/100 g water, preferably <0.15 g/100 g water, more preferably <0.10 g/100 g water;
degradation temperature at 2% weight loss of ≥350° C, preferably ≥355°C, more preferably ≥360°C;
content of metal ion of 0.1 to 10% by weight %, preferably 2 to 8 % by weight, more preferably 3 to 7 % by weight; and/or
phosphorus content of 13.5 to 14.5 weight %, preferably 13.8 to 14.3 weight %.

**9.** Use of the metal ion modified melamine polyphosphate compound of claim 1 as a flame retardant in high polymer, preferably thermoplastic or thermoset resins.

**Figure 1**

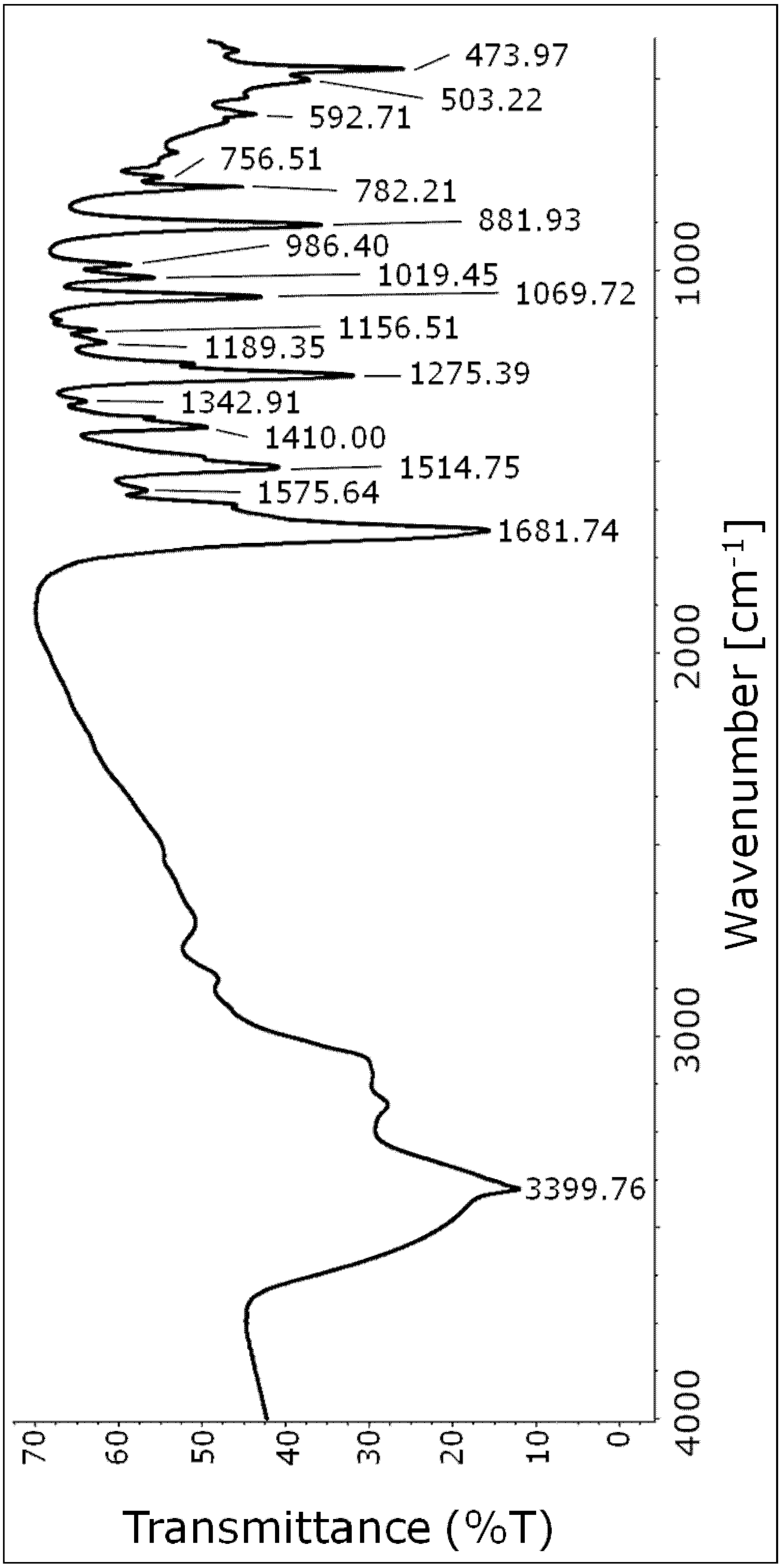
473.97
503.22
592.71
756.51
782.21
881.93
986.40
1019.45
1069.72
1156.51
1189.35
1275.39
1342.91
1410.00
1514.75
1575.64
1681.74
3399.76
1000
2000
3000
4000
Wavenumber [cm-1]
70
60
50
40
30
20
10
0
Transmittance (%T)

Figure 2

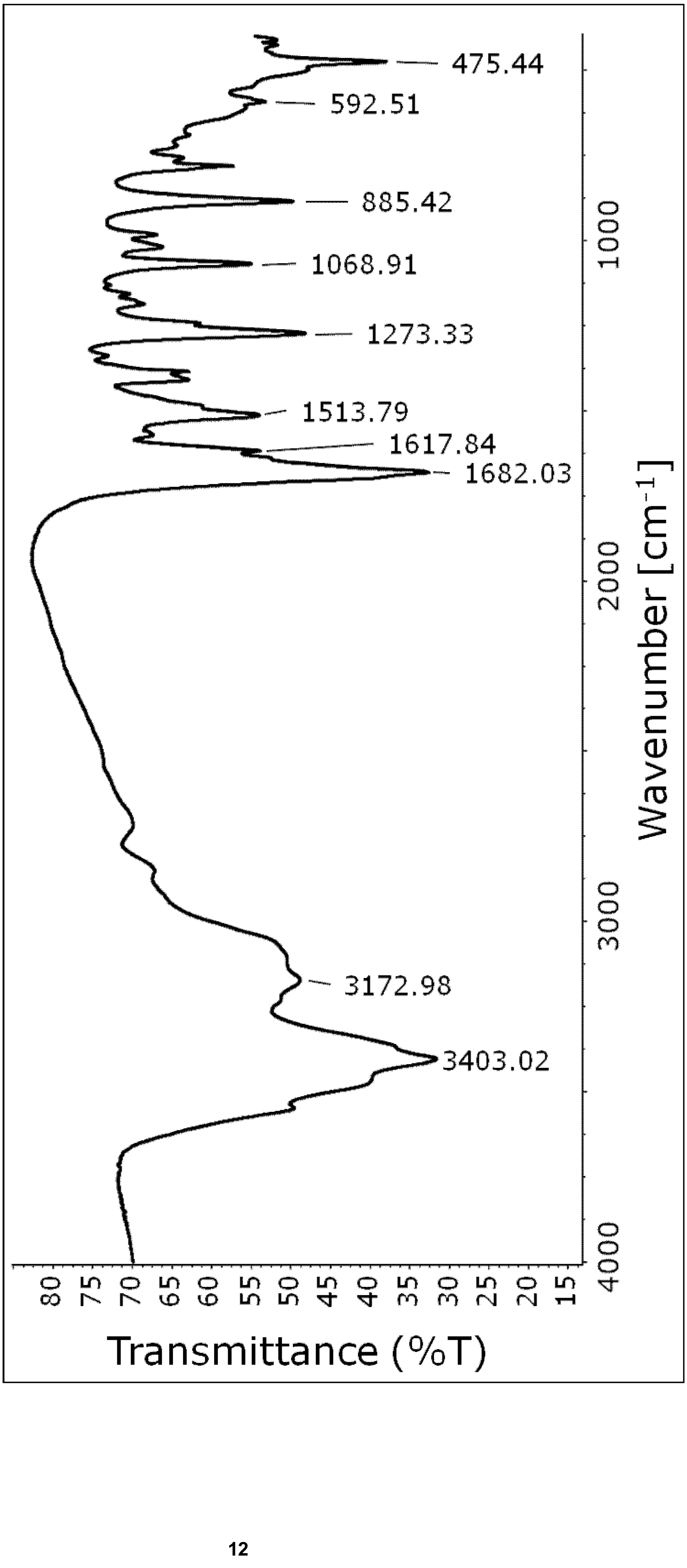
475.44
592.51
885.42
1068.91
1273.33
1513.79
1617.84
1682.03
3172.98
3403.02
1000
2000
3000
4000
Wavenumber [cm-1]
80 75 70 65 60 55 50 45 40 35 30 25 20 15
Transmittance (%T)

Europäisches Patentamt
European Patent Office
Office européen des brevets

# EUROPEAN SEARCH REPORT

Application Number
EP 17 21 0322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | R. Pfaendner, M. Döring: "Eco-friendly fire prevention, new developments in flame retardants,", Kunststoffe International 8/2014, 1 January 2014 (2014-01-01), pages 48-53, XP055473219, Retrieved from the Internet: URL:https://www.lbf.fraunhofer.de/content/dam/lbf/de/documents/AiF-Veröffentlichungen/Fachveroeffentlichungen/I-PDF%20Fraunhofer%20lbf%20KUint_2014_8%20S48ff_.pdf [retrieved on 2018-05-07] * page 49, left-hand column, paragraph 2 - page 50, left-hand column, paragraph 1; figure 2 * ----- | 1,9 | INV. C01B25/40 C09K21/00 |
| X | A.D. NAIK ET AL: "Melamine integrated metal phosphates as non-halogenated flame retardants: Synergism with aluminium phosphinate for flame retardancy in glass fiber reinforced polyamide 66", POLYMER DEGRADATION AND STABILITY, vol. 98, no. 12, 1 December 2013 (2013-12-01), pages 2653-2662, XP055116499, ISSN: 0141-3910, DOI: 10.1016/j.polymdegradstab.2013.09.029 * page 2654, right-hand column, paragraph 4; figure 1 * ----- | 1,9 | TECHNICAL FIELDS SEARCHED (IPC) C01B C09K |
| X | DE 10 2007 036465 A1 (CATENA ADDITIVES GMBH & CO KG [DE]) 5 February 2009 (2009-02-05) | 1,9 | |
| A | * paragraph [0007] - paragraph [0009] * * paragraph [0021] - paragraph [0026] * * examples 1, 6 * ----- -/-- | 2-8 | |

The present search report has been drawn up for all claims

1

| Place of search | Date of completion of the search | Examiner |
|---|---|---|
| The Hague | 8 May 2018 | Sevillano Rodriguez |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 21 0322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/137678 A9 (HUBER CORP J M [US]) 19 May 2016 (2016-05-19) * examples 5-7 * ----- | 1-9 | |
| X | US 6 008 349 A (SUZUKI KEITARO [JP] ET AL) 28 December 1999 (1999-12-28) * examples 1-5 * ----- | 1-9 | |

TECHNICAL FIELDS SEARCHED (IPC)

The present search report has been drawn up for all claims

1

| Place of search | Date of completion of the search | Examiner |
|---|---|---|
| The Hague | 8 May 2018 | Sevillano Rodriguez |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 21 0322

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2018

| Patent document cited in search report | Publication date | Patent family member(s) | Publication date |
|---|---|---|---|
| DE 102007036465 A1 | 05-02-2009 | AT 539112 T | 15-01-2012 |
| | | CN 101815751 A | 25-08-2010 |
| | | CY 1113796 T1 | 27-07-2016 |
| | | DE 102007036465 A1 | 05-02-2009 |
| | | DK 2183314 T3 | 10-04-2012 |
| | | DK 2371890 T3 | 25-03-2013 |
| | | EP 2183314 A1 | 12-05-2010 |
| | | EP 2371890 A1 | 05-10-2011 |
| | | ES 2377424 T3 | 27-03-2012 |
| | | ES 2401894 T3 | 25-04-2013 |
| | | HK 1145186 A1 | 16-05-2014 |
| | | HR P20130214 T1 | 30-06-2013 |
| | | JP 5442610 B2 | 12-03-2014 |
| | | JP 6178143 B2 | 09-08-2017 |
| | | JP 6313274 B2 | 18-04-2018 |
| | | JP 2010534692 A | 11-11-2010 |
| | | JP 2014001213 A | 09-01-2014 |
| | | JP 2016128406 A | 14-07-2016 |
| | | KR 20100052488 A | 19-05-2010 |
| | | PT 2183314 E | 16-02-2012 |
| | | PT 2371890 E | 18-03-2013 |
| | | SI 2371890 T1 | 28-06-2013 |
| | | US 2011245383 A1 | 06-10-2011 |
| | | WO 2009015772 A1 | 05-02-2009 |
| US 2016137678 A9 | 19-05-2016 | CN 104981473 A | 14-10-2015 |
| | | DK 2909215 T3 | 22-05-2017 |
| | | EP 2909215 A1 | 26-08-2015 |
| | | ES 2623944 T3 | 12-07-2017 |
| | | JP 2016502505 A | 28-01-2016 |
| | | KR 20150080519 A | 09-07-2015 |
| | | PL 2909215 T3 | 31-07-2017 |
| | | PT 2909215 T | 10-05-2017 |
| | | US 2015252065 A1 | 10-09-2015 |
| | | WO 2014060003 A1 | 24-04-2014 |
| US 6008349 A | 28-12-1999 | AT 204311 T | 15-09-2001 |
| | | AU 712183 B2 | 28-10-1999 |
| | | CA 2238474 A1 | 27-11-1997 |
| | | CN 1209824 A | 03-03-1999 |
| | | DE 69706133 D1 | 20-09-2001 |
| | | DE 69706133 T2 | 02-05-2002 |
| | | EP 0861283 A1 | 02-09-1998 |
| | | KR 100389941 B1 | 31-12-2003 |
| | | NO 983320 A | 17-07-1998 |
| | | TW 450972 B | 21-08-2001 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 17 21 0322

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2018

| Patent document cited in search report | Publication date | Patent family member(s) | Publication date |
| --- | --- | --- | --- |
| | | US 6008349 A | 28-12-1999 |
| | | WO 9744377 A1 | 27-11-1997 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101014645 A
- CN 1314898 A
- WO 9744377 A
- CN 13624138
- EP 1789475 B1
- CN 1733778 A
- EP 1040754 A2
- US 20070299171 A


### Non-patent literature cited in the description


- **GORDON L. NELSON.** Fire and Polymers. 05 September 1990, 228-231
- Polyphosphate Flame Retardants with Increased Heat Resistance. *Ind. Eng. Chem. Res.,* 31 May 2003
- **GORDON L. NELSON.** Fire and Polymers. 09 May 1990
- **OU YU XIANG.** Combustion Behavior and Thermal Pyrolysis of PA6 Flame Retarded with Melamine Polyphosphate Based Intumescent Flame Retardants. *China Plastics,* 2003